# EUROPÄISCHE PATENTSCHRIFT

(11) **EP 2 665 455 B1**
(45) Veröffentlichungstag und Bekanntmachung des Hinweises auf die Patenterteilung: **27.04.2016**
(21) Anmeldenummer: 12700323.4
(22) Anmeldetag: 04.01.2012
(51) Int. Cl.: A61F 13/15, A61F 13/496

(54) **VERFAHREN ZUM HERSTELLEN ABSORBIERENDER HYGIENEARTIKEL**
METHOD FOR PRODUCING AN ABSORBENT PERSONAL HYGIENE PRODUCT
PROCÉDÉ POUR PRODUIRE UN ARTICLE HYGIÉNIQUE

(30) Priorität: 19.01.2011 DE 102011009549
(43) Veröffentlichungstag der Anmeldung: 27.11.2013
(73) Patentinhaber: Paul Hartmann AG, 89522 Heidenheim (DE)
(72) Erfinder: GASSNER, Oliver, 50674 Köln (DE); OSTERTAG, Wolfgang, 89547 Gerstetten (DE); BEYRLE, Andreas, 89564 Nattheim (DE); ZIEGLER, Danilo, 73179 Ellwangen/Jagst (DE)
(74) Vertreter: DREISS Patentanwälte PartG mbB
(86) Internationale Anmeldenummer: PCT/EP2012/050095
(87) Internationale Veröffentlichungsnummer: WO 2012/098013

(56) Entgegenhaltungen:
- DE-A1- 4 004 879
- US-A- 3 730 798
- US-A- 5 932 284
- US-A1- 2008 287 898

## Beschreibung

Die Erfindung betrifft ein Verfahren zum Herstellen absorbierender Hygieneartikel zum einmaligen Gebrauch, insbesondere in Höschenform, in endloser Fertigung in einer schnelllaufenden Herstellungsmaschine, wobei Flachmaterialabschnitte durch Sprühkleberauftrag mittels Sprühdüsen miteinander gefügt und verklebt werden, wobei der Sprühkleber auf eine in einer Maschinenrichtung geführte Bahn gesprüht wird.

Wenn im Zuge der Herstellung eines absorbierenden Hygieneartikels Flachmaterialabschnitte miteinander verklebt werden, so stellt sich das Problem, dass eine innige bis zum Rand eines Flachmaterialabschnitts ausgebildete Kleberverbindung nur dann erzielbar ist, wenn dieser Flachmaterialabschnitt zuvor auch bis zum betreffenden Rand mit Sprühkleber beaufschlagt wurde. Da in schnelllaufenden Herstellungsmaschinen, die mit hohen Maschinengeschwindigkeiten, insbesondere mit wenigstens 100 m/min, insbesondere mit wenigstens 200 m/min oder darüber geförderten Bahnen jedoch in der Querrichtung nie ganz exakt geführt werden können, ist die Herstellung einer Kleberbeschichtung exakt bis zum Rand einer Bahn oder eines Flachmaterialabschnitts der Bahn nicht stets möglich, was - wie erwähnt - zu dem Problem führt, dass der betreffende Flachmaterialabschnitt nicht flächenhaft bis zum Rand mit einem anderen Flachmaterialabschnitt verklebt werden kann. Es besteht solchenfalls das Problem oder die Gefahr, dass diese nicht verklebten Ränder sich im Gebrauch des Hygieneartikels abheben und zu einem unangenehmen Tragegefühl und möglicherweise zu Hautirritationen führen. Ein Sprühkleberauftrag, der prozesssicher den gesamten Rand einer zu beschichtenden Bahn oder eines zu beschichtenden Flachmaterialabschnitts der Bahn erfasst, würde auch bei genauer Ausrichtung der Sprühdüse infolge der variablen Querführung der Bahn dazu führen, dass Sprühkleber die Umgebung der Auftragsstelle innerhalb der Herstellungsmaschine unkontrolliert verunreinigt, was mit erheblichen Nachteilen verbunden ist, da Kleberverschmutzungen innerhalb der Maschine nur sehr zeitaufwändig wieder entfernt werden können und außerdem die Prozesssicherheit und die Standzeit eines Produktionszyklus beeinträchtigen.

Beispielsweise stellt sich dieses Problem bei der Herstellung von Hygieneartikeln in Höschenform, wie sie insbesondere aus US 2008/0287898 A1 bekannt sind, bei denen ein Schrittabschnitt mit einem vorderen Bauchabschnitt und einem hinteren Rückenabschnitt, die im Zuge der Herstellung jeweils in Form einer endlosen Bahn gefördert werden, in Überlappung gebracht und dann verklebt werden. Auch in diesem Fall ist es höchst wünschenswert, dass die Längsrandabschnitte des Schrittabschnitts mit dem Bauchabschnitt bzw. mit dem Rückenabschnitt randbündig verklebt wird, so dass es nicht zum Aufwerfen von Rändern oder Randbereichen kommt. Unkontrolliert abstehende Ränder oder Randbereiche des Schrittabschnitts, wie die des herkömmlicherweise auf Folien basierenden Backsheets, können zu unkontrollierter Faltenbildung und Hartstellen und nachteiligerweise zu einem unangenehmen Tragegefühl und Hautirritationen führen. Randbündiges Verkleben war aber seither nicht möglich.

US 5,932,284 zeigt ein gattungsgemäßes Verfahren.

Der vorliegenden Erfindung liegt die Aufgabe zugrunde, ein Verfahren der eingangs genannten Art dahingehend zu verbessern, dass in prozesssicherer und wirtschaftlicher Weise eine optimale Kleberverbindung auch in einem Randbereich von miteinander zu fügenden Flachmaterialabschnitten realisierbar ist.

Diese Aufgabe wird durch ein Verfahren mit den Merkmalen des Anspruchs 1 gelöst.

Nach der Erfindung wird also eine randständige, d.h. den Rand der Bahn erfassende bzw. bis zum Rand der Bahn erstreckte Sprühkleberbeschichtung auch bei mehr oder weniger ausgeprägter Variation in der Bahnquerführung dadurch erreicht, dass der sich insbesondere erweiternde Sprühstrahl so bezüglich des Rands der Bahn eingestellt wird, dass bewusst ein Teil des Sprühklebers an dem Rand der Bahn vorbeigesprüht wird. Hierdurch lässt sich nämlich prozesssicher eine randständige Beleimung der Bahn oder eines Flachmaterialabschnitts der Bahn erreichen. Um eine Verunreinigung der Umgebung innerhalb der Herstellungsmaschine zu vermeiden, wird in dem betreffenden Förderabschnitt, in dem der Sprühkleberauftrag ausgeführt wird, vorzugsweise in Überlappung mit der Bahn und in geringem Abstand zu der Bahnebene, das erwähnte Auffangmittel für den an der Bahn vorbeigesprühten Teil des Sprühklebers vorgesehen. Das Auffangmittel dient also der gezielten Aufnahme, Sammlung und Abführung des an der Bahn vorbeigesprühten Sprühklebers.

Vorzugsweise wird das Auffangmittel in Überlappung mit der Bahn hinter die Bahnebene, also auf die der Sprühdüse abgewandten Seite der Bahn, reichend oder sich erstreckend vorgesehen, damit jeglicher Sprühkleber, der an dem Rand der Bahn vorbeigesprüht wird, sicher aufgefangen wird, auch wenn die Bahn in der Querrichtung nicht exakt geführt wird.

Um den Sprühkleber aus der Herstellungsmaschine abzuführen und gegebenenfalls einer Wiederverwendung zuzuführen, erweist es sich als vorteilhaft, dass das Auffangmittel flächenhaft ausgebildet und beheizt ist, insbesondere von einem beheizten Blech gebildet ist, so dass der Sprühkleber im flüssigen Zustand von dem Auffangmittel abgeführt werden kann. Zweckmäßigerweise kann bei dem flächenhaften Auffangmittel ein natürliches Gefälle im Raum vorgesehen sein, entlang dessen der Kleber ablaufen kann.

Nach einer anderen Alternative der Erfindung wird das Auffangmittel von einer beheizten Führungs- oder Umlenkrolle gebildet, so dass auch hier der Sprühkleber im flüssigen Zustand von dem Auffangmittel abgeführt werden kann, was durch die Rotation der Umlenkrolle noch begünstigt werden kann.

Es erweist sich für das erfindungsgemäße Verfahren als vorteilhaft, wenn der Sprühkleberauftrag in einem Förderabschnitt der Maschine ausgeführt wird, in dem die Bahn zumindest randseitig nicht von oben oder unten geführt ist, so dass dort das Auffangmittel nahe bei der Bahn in geringem Abstand zu der Bahn und vorzugsweise hinter die Bahnebene reichend positioniert werden kann. Es sei an dieser Stelle darauf hingewiesen, dass der erwähnte geringe Abstand im Bereich von 0,5 mm bis wenige cm liegen kann. Selbstverständlich wäre ein Auffangen des an der Bahn vorbeigesprühten Sprühklebers auch in einem Abstand von beispielsweise 10 cm oder 15 cm oder sogar darüber möglich, jedoch würde solchenfalls insgesamt mehr Kleber mit der Luft innerhalb der Maschine verwirbelt werden und möglicherweise nicht abgeschieden und abgeführt werden können. Vor diesem Hintergrund erweist es sich als vorteilhaft, wenn das Auffangmittel vorzugsweise möglichst nahe bei dem mit Sprühkleber zu beschichtenden Bahnabschnitt vorgesehen wird. Wie eingangs bereits angedeutet, kann das erfindungsgemäße Verfahren Anwendung finden bei der Herstellung von Hygieneartikeln in Höschenform, die mit einem vorderen Bauchabschnitt und einem hinteren Rückenabschnitt ausgebildet sind, die zur Bildung einer durchgehenden Hüftöffnung an beidseitigen Seitennahtbereichen herstellerseitig miteinander verbunden werden, und die ferner einen Schrittabschnitt aufweisen, der sich in einer Längsrichtung des Hygieneartikels zwischen dem Bauchabschnitt und dem Rückenabschnitt erstreckt und diese überlappt, so dass er im Überlappungsbereich an den Bauchabschnitt und an den Rückenabschnitt mittels Sprühkleber unlösbar angefügt werden kann. Solchenfalls bilden der Schrittabschnitt, der Bauchabschnitt und der Rückenabschnitt die zu fügenden Materialabschnitte, wobei die in der Maschinenrichtung geführte und randseitig unter Ausführung des erfindungsgemäßen Verfahrens mit Sprühkleber beaufschlagte Bahn die späteren Schrittabschnitte des herzustellenden Hygieneartikels bildet.

Im Zuge des Ausführung des Verfahrens zur Herstellung derartiger vorgenannter Hygieneartikel in Höschenform erweist es sich als vorteilhaft, wenn die die Schrittabschnitte bildende Bahn nach dem Aufsprühen von Sprühkleber quer zur Maschinenrichtung geschnitten wird, so dass aufeinanderfolgende vereinzelte Schrittabschnitte gebildet werden, die dann um 90 Grad gedreht und in Überlappung mit einer die Rückenabschnitte und einer die Bauchabschnitte bildenden und in der Maschinenrichtung geförderten Bahn gebracht und mit diesen klebend verbunden werden.

Die Erfindung betrifft ferner einen Hygieneartikel in Höschenform mit den Merkmalen des Anspruchs 7. Dadurch, dass die Sprühkleberverbindung in Querrichtung bis zum äußersten Längsrand des Schrittabschnitts im Überlappungsbereich mit dem Bauchabschnitt und mit dem Rückenabschnitt ausgebildet oder erstreckt ist, bleiben die Längsränder des Schrittabschnitts gegen den Bauchabschnitt und gegen den Rückenabschnitt fixiert. Es kommt also nicht zu aufstehenden Längsrändern, die zu Hautirritationen führen können. Es zeigt sich außerdem, dass hierdurch eine Verbesserung des Verbunds zwischen Schrittabschnitt und Bauchabschnitt bzw. Schrittabschnitt und Rückenabschnitt erreicht ist. Insbesondere beim Anziehen der Windelhose ergeben sich nämlich erhebliche schräg nach oben wirkende Zugkräfte, die durch den Benutzer in die Chassismaterialien der Windelhose eingeleitet werden. Hierdurch wird die Fügeverbindung zwischen Schrittabschnitt und Bauchabschnitt sowie Rückenabschnitt stark beansprucht. Diese Beanspruchung führte seither in verstärktem Maße zu den vorausgehend angesprochenen sich abhebenden Längsrändern des Schrittabschnitts und den hiermit verbundenen Folgen. Auch die in Umfangsrichtung verlaufenden Elastifizierungsmittel, die bei Windelhosen gewissermaßen über den gesamten Bauch- und Rückenabschnitt verteilt angeordnet sind, üben auf die Fügeverbindung von Schrittabschnitt und Bauchabschnitt bzw. Rückenabschnitt eine ständige Belastung aus. Insbesondere im oberen, also in Längsrichtung hinteren und vorderen Längsrandende des Schrittsabschnitts gestalten sich diese Randprobleme als besonders problematisch. Durch die erfindungsgemäße Ausbildung der Sprühkleberverbindung dergestalt, dass sich der Kleber im Überlappungsbereich bis zum äußeren Rand des Schrittabschnitts erstreckt, kann eine bis zum äußeren Rand wirksame Kleberverbindung zwischen Schrittabschnitt und Bauchabschnitt und Rückenabschnitt hergestellt werden, welche sich auf die vorstehend beschriebene Beanspruchungssituation positiv und stabilisierend auswirkt.

Desweiteren ist Gegenstand der Erfindung eine Vorrichtung zur Durchführung des erfindungsgemäßen Verfahrens mit den Merkmalen des Anspruchs 8. Insbesondere erweist es sich als vorteilhaft, wenn das Auffangmittel in Überlappung mit der Bahn hinter die Bahnebene reichend vorgesehen ist.

Erfindungsgemäß erweist es sich als vorteilhaft, dass das Auffangmittel flächenhaft ausgebildet und beheizt ist, insbesondere von einem beheizten Blech gebildet ist, so dass der Sprühkleber im flüssigen Zustand von dem Auffangmittel abgeführt werden kann.

Erfindungsgemäß erweist es sich als vorteilhaft, dass das Auffangmittel von einer beheizten Führungs- oder Umlenkrolle gebildet ist, so dass der Sprühkleber im flüssigen Zustand von dem Auffangmittel abgeführt werden kann.

Insbesondere erweist es sich als vorteilhaft, wenn die Bahn in einem Förderabschnitt der Maschine, in dem der Sprühkleberauftrag ausgeführt wird, zumindest randseitig nicht geführt ist.

In der Zeichnung zeigt:
- Figuren 1 und 2: eine schematische Darstellung einer ersten und einer zweiten Ausführungsform des erfindungsgemäßen Verfahrens in zwei Varianten;
- Figuren 3a, 3b: eine dritte nicht erfindungsgemäße Ausführungsform des erfindungsgemäßen Verfahrens;
- Figur 4: eine Draufsicht auf einen Hygieneartikel, der unter Ausführung des erfindungsgemäßen Verfahrens hergestellt wird;
- Figur 5: eine schematische Seitenansicht im Bereich eines Förderabschnitts einer Maschine, in dem eine nicht erfindungsgemäße Sprühkleberbeschichtung ausgeführt wird; und
- Figur 6: eine schematische Seitenansicht des Förderabschnitts nach Figur 5 mit einer daran anschließenden Vereinzelungsstation mit Dreheinheit und Applikation auf eine weitere Bahn.

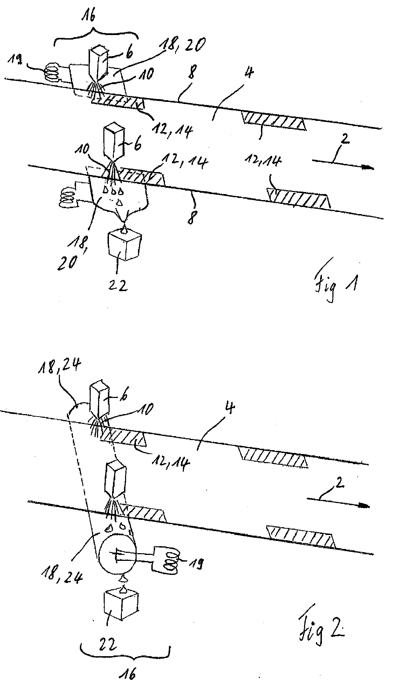

Die Figuren 1 und 2 zeigen schematisch eine in erfindungsgemäßer Weise ausgeführte Sprühkleberbeschichtung bei einer in einer Maschinenrichtung 2 endlos geführten Bahn 4 in einer schnelllaufenden Maschine zur Herstellung absorbierender Hygieneartikel. Diese Bahn 4 wird an späterer Stelle quer zur Maschinenrichtung 2 geschnitten, wodurch Flachmaterialabschnitte gebildet werden, die mit weiteren Flachmaterialabschnitten oder Abschnitten weiterer in endloser Richtung geführter Bahnen mittels Kleber unlösbar gefügt werden. Die Figuren 1 und 2 zeigen schematisch Sprühdüsen 6, die erfindungsgemäß so in Bezug auf jeweilige Ränder 8 der Bahn 4 positioniert und ausgerichtet sind, dass ein sich insbesondere kegelförmig erweiternder Sprühstrahl 10 der jeweiligen Sprühdüse 6 einen in der Maschinenrichtung 2 erstreckten Längsrandabschnitt 12 der Bahn 4 mit Sprühkleber intermittierend beaufschlagt, so dass in der Maschinenrichtung 2 aufeinander folgend und voneinander beabstandet erstreckte Kleberspuren 14 gebildet werden, die die Längsränder 8 also bis zur Randkante erfassen. Erfindungsgemäß erstreckt sich der jeweilige insbesondere kegelförmige Sprühstrahl 10 der betreffenden Sprühdüse 6 über den Rand 8 der Bahn 4 hinaus, so dass ein Teil des Sprühklebers an der Bahn vorbeigesprüht wird. Auf diese Weise wird sichergestellt, dass die Bahn 4 wirklich randständig, d. h. bis zum äußersten Längsrand 8 der Bahn 4 mit Sprühkleber beschichtet wird.

Um zu verhindern, dass der an der Bahn 4 vorbeigesprühte Teil des Sprühklebers unkontrolliert die Umgebung der Herstellungsmaschine beaufschlagt und verunreinigt, wird in demjenigen Förderabschnitt 16 der Maschine, wo der Sprühkleberauftrag ausgeführt wird, ein Auffangmittel 18 für den über den Rand 8 der Bahn 4 hinausgehenden Teil des Sprühklebers vorgesehen. Im beispielhaft dargestellten Fall der Figur 1 ist das Auffangmittel 18 flächenhaft ausgebildet und beheizt; es ist insbesondere von einem beheizten Blech 20 gebildet. Eine schematisch dargestellte Heizeinrichtung ist mit Bezugszeichen 19 bezeichnet. Das beheizte Blech 20 ist beispielhaft derart geformt und/oder geneigt angeordnet, dass der von dem Blech 20 aufgefangene Sprühkleber zu einer Sammelvorrichtung 22 ablaufen kann, die ebenfalls nur schematisch dargestellt ist. Der dort gesammelte Sprühkleber kann entweder entsorgt oder einer Wiederverwendung zugeführt werden.

Bei der in Figur 2 dargestellten Ausführungsform ist das Auffangmittel 18 von einer beheizten Führungs- oder Umlenkrolle 24 gebildet, an der sich ebenfalls an der Bahn vorbeigesprühter Sprühkleber niederschlägt und von dort in die Sammelvorrichtung 22 ablaufen kann.

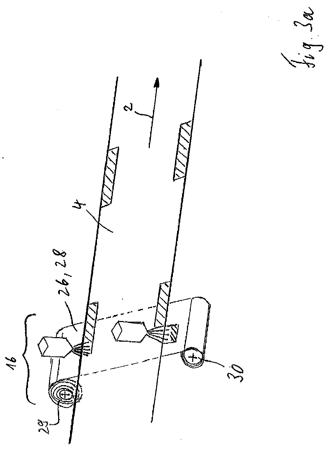

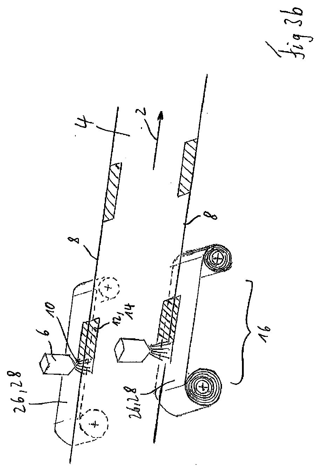

Die Figuren 3a und 3b zeigen ein nicht erfindungsgemäßes Auffangmittel 26 in Form eines biegsamen Flachmaterials 28 verwendet wird, um überschüssigen Sprühkleber, der an der Bahn 4 vorbeigesprüht wird, aufzufangen. Das Flachmaterial 28 kann beispielsweise von einem Vlies, einer Tissuebahn oder einem textilen Flachmaterial gebildet sein. Es ist im bevorzugten dargestellten Fall endlos vorgesehen, so dass es dem Förderabschnitt 16, wo der Sprühkleberauftrag ausgeführt wird, kontinuierlich oder diskontinuierlich zugeführt bzw. von dort abgeführt werden kann. Hierfür wird das Flachmaterial 28 von einer ersten Rolle 29 abgerollt, um dem Förderabschnitt 16 zugeführt zu werden; es wird auf eine zweite Rolle 30 aufgerollt, um aus dem Förderabschnitt 16 abgeführt zu werden. Insgesamt kann also kontinuierlich oder diskontinuierlich, insbesondere zyklisch und vorzugsweise automatisiert gesteuert frisches Flachmaterial 28 dem Förderabschnitt 16 zugeführt und mit Sprühkleber beaufschlagtes Flachmaterial 28 aus dem Förderabschnitt 16 abgeführt werden. Auf diese Weise lässt sich der an der Bahn 4 vorbeigesprühte Sprühkleber prozesssicher aus einer Umgebung des Förderabschnitts 16 bzw. der Herstellungsmaschine entfernen. Vorzugsweise ist das Auffangmittel 18 bzw. 26 in vorzugsweise geringem Abstand hinter der Bahnebene der Bahn 4 vorgesehen bzw. geführt, um sicherzustellen, dass möglichst der gesamte Sprühkleber, der nicht auf den Längsrandabschnitt 12 gelangt, von dem Auffangmittel 18, 26 aufgenommen wird. Bei der Variante gemäß Figur 3a wird das Entfernen und Erneuern des Auffangmittels 26, d. h. das Zuführen und Abführen des biegsamen Flachmaterials 28 quer zur Maschinenrichtung 2 ausgeführt. Bei der Verfahrensvariante der Figur 3b ist im Bereich jedes Längsrands 8 und nahe der Bahn 4 ein entsprechendes Auffangmittel 26 vorgesehen, das jedoch in der Maschinenrichtung 2 entfernt und erneuert, d. h. zugeführt und abgeführt wird. Diese Ausführungsform kann sich beispielsweise als vorteilhaft erweisen, wenn im Bereich unterhalb der Mitte der Bahn 4 kein Raum zur Verfügung steht, um das Auffangmittel 26 in Querrichtung unter der Bahn, wie bei Figur 3a, hindurchzuführen, etwa weil dort Stütz- und Führungsmittel für die Bahn vorgesehen werden müssen.

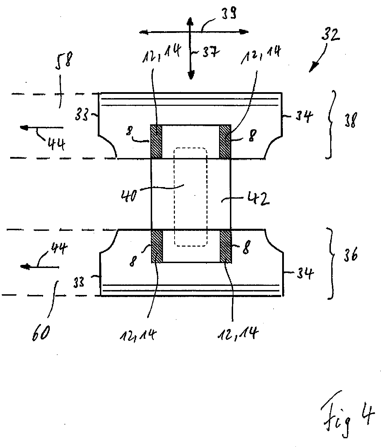

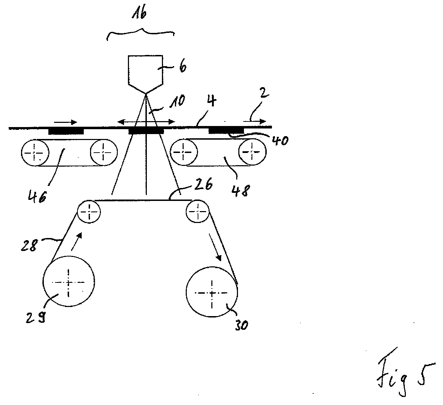

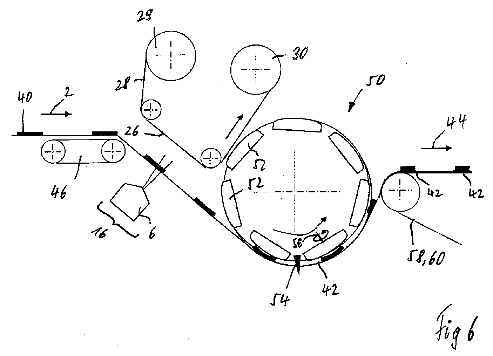

Figur 4 zeigt schematisch eine Draufsicht auf einen Hygieneartikel 32, der nach dem Fügen von Seitennahtbereichen 33, 34 eine Höschenform annimmt. Der Hygieneartikel 32 umfasst einen vorderen Bauchabschnitt 36 und einen hinteren Rückenabschnitt 38, die zur Bildung einer in Hüftumfangsrichtung durchgehenden Hüftöffnung an den beidseitigen Seitennahtbereichen 33 und 34 miteinander herstellerseitig unlösbar gefügt werden. Eine Längsrichtung des Hygieneartikels 32 trägt das Bezugszeichen 37 und eine Querrichtung das Bezugszeichen 39. Der Hygieneartikel 32 umfasst ferner einen einen Absorptionskörper 40 aufweisenden Schrittabschnitt 42, der über die zuvor im Zusammenhang mit den Figuren 1 bis 3 erörterten Kleberspuren 14 in Längsrandabschnitten 12 mit dem jeweiligen Bauchabschnitt 36 und Rückenabschnitt 38 unlösbar gefügt ist. Durch den vorausgehend beschriebenen Sprühkleberauftrag erstreckt sich die Kleberspur 14 in Querrichtung 39 des Hygieneartikels 32 bis zum äußeren Längsrand 8 des Schrittabschnitts 42, so dass eine Kleberverbindung mit dem Bauchabschnitt 36 und dem Rückenabschnitt 38 bis zum äußeren Längsrand 8 des Schrittabschnitts 42 erreicht wird. Hierfür wird ein jeweiliger Schrittabschnitt 42 des Hygieneartikels 32 von der in den Figuren 1 bis 3 erörterten endlosen Bahn 4 senkrecht zur Maschinenrichtung 2, abgetrennt. Ein solcher jeweiliger Schrittabschnitt 42 wird dann um 90° um eine auf der Bahnebene senkrecht stehende Achse gedreht und dann in Superposition auf eine die Bauchabschnitte 36 und eine die Rückenabschnitte 38 bildende endlose Bahn gebracht, die in einer Maschinenrichtung 44 gefördert wird, was in Bezug auf die in Figur 4 dargestellten Komponenten des Hygieneartikels 30 mit Bezugszeichen 44, 58, 60 angedeutet ist. Dies wird im Zusammenhang mit Figur 6 erläutert werden. Zuvor zeigt Figur 5 eine schematische Darstellung der Sprühkleberapplikation in dem Förderabschnitt 16. Mit Bezugszeichen 4 ist diejenige die späteren Schrittabschnitte 42 von Figur 4 bildende Bahn bezeichnet. Bezugszeichen 40 bezeichnet an sich übliche Absorptionskörper bei Hygieneartikeln. Führungsvorrichtungen 46, 48 in Form von Unterdruckführungsbändern sind schematisch dargestellt. Die Bahn 4 liegt mit ihrer die spätere körperzugewandete Seite der Hygieneartikel bildenden Seite gegen die Führungsvorrichtungen 46, 48 an. Bei der Bahn 4 handelt es sich beispielsweise um eine das spätere Backsheet des Hygieneartikels bildende Flachmaterialbahn. In dem Förderabschnitt 16 werden die aus den vorherigen Figuren ersichtlichen Längsrandabschnitte 12 randständig besprüht, wobei überschüssiger Sprühkleber durch das Auffangmittel 26 aufgefangen ist, das entsprechend der Figur 3b ausgebildet ist.

Figur 6 zeigt eine entsprechende Flachmaterialbahn 4 mit Absorptionskörpern 40, die in der Maschinenrichtung 2 endlos gefördert wird. Sie liegt im Unterschied zu der Darstellung nach Figur 5 mit der späteren körperabgewandten Seite gegen Führungsvorrichtung 46 an. In einem Förderabschnitt 16 der Maschine, wo die Bahn 4 nicht gestützt ist, wo also vorzugsweise keine Führungsvorrichtungen 46 für die Bahn vorgesehen sind, erfolgt der Sprühkleberauftrag wie im Zusammenhang mit Figur 5 beschrieben, wobei entsprechende Komponenten entsprechende Bezugszeichen tragen. Der Sprühkleberstation nachgeordnet ist eine rotierende Vorrichtung 50 mit einer Mehrzahl von Schuhen 52. Die Bahn 4 mit sprühkleberbeschichteten Längsrandabschnitten 12 wird dieser rotierenden Vorrichtung 50 zugeführt, wobei die Bahn 4 mit ihrer späteren körperzugewandten Seite gegen die rotierende Vorrichtung 50 bzw. gegen die unterdruckbeaufschlagbaren Schuhe 52 angelegt und von dieser Vorrichtung 50 weiter transportiert wird. Mit Bezugszeichen 54 ist eine Querschneidvorrichtung angedeutet, mittels derer Längsabschnitte von der Bahn 4 abgetrennt werden, welche die in Figur 4 dargestellten Schrittabschnitte 42 bilden. Ein jeweiliger Schuh 52 ist um eine radiale Achse 56 um 90° drehbar, so dass ein jeweiliger Schrittabschnitt 42 um 90° bezüglich der Bahnebene und der Maschinenrichtung 2 gedreht wird. Er wird dann nicht mehr in seiner Längsrichtung sondern in seiner Querrichtung 39 (bezogen auf den herzustellenden Hygieneartikel) weiter gefördert. Im Weiteren erfolgt eine Superposition mit einer die Bauchabschnitte 36 und einer die Rückenabschnitte 38 bildenden endlosen Bahn 58 und 60 (die auch schematisch in Figur 4 angedeutet sind). Ein jeweiliger Schrittabschnitt 42 wird dabei mit seinen sprühkleberbeschichteten Längsrandabschnitten 12 in Überlappung mit den Bahnen 58, 60 gebracht und daran klebend fixiert. Durch die beschriebene Verfahrensführung wird erfindungsgemäß erreicht, dass ein jeweiliger Schrittabschnitt 42 infolge der bis zu seinem Rand 8 erstreckten Kleberbeschichtung flächenhaft bis zum Rand gegen den betreffenden Bauchabschnitt 36 und Rückenabschnitt 38 fixiert ist, so dass es nicht zu aufstehenden Längsrändern 8 bei dem Hygieneartikel kommt. Im Anschluss an den in Figur 6 rechts dargestellten Fügeprozess wird der Verbund aus endlosen Bahnen 58, 60 und diese überbrückenden Schrittabschnitten 42 in nicht dargestellter, jedoch an sich bekannter Weise um die Maschinenrichtung auf sich selbst gefaltet und an den Seitennahtbereichen 33, 34 miteinander unlösbar fixiert und schließlich durch einen weiteren Trennvorgang in quer zur Maschinenrichtung vereinzelt, was jedoch an sich bekannt ist und daher keiner weiteren Beschreibung bedarf.

## Patentansprüche

1. Verfahren zum Herstellen absorbierender Hygieneartikel (32) zum einmaligen Gebrauch, insbesondere in Höschenform, in endloser Fertigung in einer schnelllaufenden Herstellungsmaschine, wobei Flachmaterialabschnitte durch Sprühkleberauftrag mittels Sprühdüsen (6) miteinander gefügt und verklebt werden, wobei der Sprühkleber (3) auf eine in einer Maschinenrichtung (2) geführte Bahn (4) gesprüht wird, wobei zur Bildung eines einen Rand (8) der Bahn (4) erfassenden Sprühkleberauftrags die Sprühdüse (6) in einem Förderabschnitt (16) der Maschine so in Bezug auf den Rand (8) der Bahn (4) positioniert und ausgerichtet wird, dass ihr insbesondere kegel- oder fächerförmig sich erweiternder Sprühstrahl (10) über den Rand (8) der Bahn (4) hinausreicht, so dass die Bahn (4) bis zum Rand (8) mit Sprühkleber besprüht wird und ein Teil des Sprühklebers an der Bahn (4) vorbei gesprüht wird, und wobei in diesem Förderabschnitt (16) in vorzugsweise geringem Abstand zu einer Ebene der Bahn (4) ein den Sprühstrahl (10) erfassendes Auffangmittel (18, 26) für den über den Rand (8) der Bahn (4) hinausgehenden Teil des Sprühklebers vorgesehen ist, so dass an dem Rand (8) der Bahn (4) vorbei gesprühter Sprühkleber nicht zu Verunreinigungen der Maschine führt, **dadurch gekennzeichnet, dass** das Auffangmittel (18) flächenhaft ausgebildet und beheizt ist oder von einer beheizten Führungs- oder Umlenkrolle (24) gebildet ist, so dass der Sprühkleber im flüssigen Zustand von dem Auffangmittel (18) abgeführt wird.

2. Verfahren nach Anspruch 1, **dadurch gekennzeichnet, dass** das Auffangmittel (18, 26) in Überlappung mit der Bahn (4) hinter die Bahnebene reichend vorgesehen wird.

3. Verfahren nach Anspruch 1 oder 2, **dadurch gekennzeichnet, dass** das Auffangmittel (18) von einem beheizten Blech (20) gebildet ist.

4. Verfahren nach einem oder mehreren der vorstehenden Ansprüche, **dadurch gekennzeichnet, dass** der Sprühkleberauftrag in einem Förderabschnitt (16) der Maschine ausgeführt wird, in dem die Bahn (4) zumindest randseitig nicht geführt ist.

5. Verfahren nach einem oder mehreren der vorstehenden Ansprüche, **dadurch gekennzeichnet, dass** der Hygieneartikel (32) in Höschenform und mit einem vorderen Bauchabschnitt (36) und einem hinteren Rückenabschnitt (38) ausgebildet ist, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen (33, 34) herstellerseitig miteinander verbunden werden, und mit einem einen Absorptionskörper (40) aufweisenden Schrittabschnitt (42), der sich in einer Längsrichtung zwischen Bauchabschnitt (36) und Rückenabschnitt (38) erstreckt und diese überlappt, und dass er im Überlappungsbereich an den Bauchabschnitt (36) und an den Rückenabschnitt (38) mittels Sprühkleber unlösbar angefügt wird, wobei der Schrittabschnitt (42), der Bauchabschnitt (36) und der Rückenabschnitt (38) die zu fügenden Materialabschnitte bilden und die in der Maschinenrichtung (2) geführte und randseitig mit Sprühkleber beaufschlagte Bahn (4) die Schrittabschnitte (42) bildet.

6. Verfahren nach Anspruch 5, **dadurch gekennzeichnet, dass** die die Schrittabschnitte (42) bildende Bahn (4) nach dem Aufsprühen von Sprühkleber quer zur Maschinenrichtung (2) geschnitten wird, so dass aufeinanderfolgende vereinzelte Schrittabschnitte (42) gebildet werden, die dann um 90 Grad gedreht und in Überlappung mit einer die Rückenabschnitte (36) und einer die Bauchabschnitte (38) bildenden und in einer Maschinenrichtung (44) geförderten Bahn (58, 60) gebracht und mit diesen klebend verbunden werden.

7. Hygieneartikel (32) in Höschenform, mit einem vorderen Bauchabschnitt (36) und einem hinteren Rückenabschnitt (38), die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen (33, 34) herstellerseitig miteinander verbunden sind, und mit einem einen Absorptionskörper (40) aufweisenden Schrittabschnitt (42), der sich in einer Längsrichtung (37) zwischen Bauchabschnitt (36) und Rückenabschnitt (38) erstreckt und diese überlapptund im Überlappungsbereich an den Bauchabschnitt (36) und an den Rückenabschnitt (38) mittels Sprühkleber unlösbar angefügt ist, wobei die Sprühkleberverbindung zwischen einem Längsrandabschnitt (12) des Schrittabschnitts (42) und dem Bauchabschnitt (36) und Rückenabschnitt (38) ausgebildet ist, erhalten durch ein Verfahren mit den Merkmalen des Oberbegriffs des Anspruchs 1 und **dadurch gekennzeichnet, dass** die Sprühkleberverbindung in Querrichtung (39) bis zum äußersten Längsrand (8) des Schrittabschnitts (42) ausgebildet ist, indem die in der Maschinenrichtung (2) geführte und randseitig mit Sprühkleber beaufschlagte Bahn (4) die Schrittabschnitte (42) bildet, so dass sich keine Längsränder (8) des Schrittabschnitts (42) von dem Bauchabschnitt (36) oder Rückenabschnitt (38) emporheben und zu Hautirritationen führen können.

8. Vorrichtung zur Durchführung des Verfahrens nach einem oder mehreren der vorstehenden Ansprüche 1-6,wobei in einem Förderabschnitt der schnelllaufenden Herstellungsmaschine, in dem der Sprühkleberauftrag ausgeführt wird, eine Sprühdüse (6) so in Bezug auf den Rand (8) der zu besprühenden Bahn (4) positioniert und ausgerichtet wird, dass ihr insbesondere kegel- oder fächerförmig sich erweiternder Sprühstrahl (10) über den Rand (8) der Bahn (4) hinausreicht, so dass die Bahn (4) bis zum Rand (8) mit Sprühkleber besprüht wird und ein Teil des Sprühklebers an der Bahn (4) vorbei gesprüht wird, und wobei in diesem Förderabschnitt (16) in vorzugsweise geringem Abstand zu einer Ebene der Bahn (4) ein den Sprühstrahl (10) erfassendes Auffangmittel (18, 26) für den über den Rand (8) der Bahn (4) hinausgehenden Teil des Sprühklebers vorgesehen ist, so dass an dem Rand (8) der Bahn (4) vorbei gesprühter Sprühkleber nicht zu Verunreinigungen der Maschine führt, **dadurch gekennzeichnet, dass** das Auffangmittel (18) flächenhaft ausgebildet und beheizt ist oder von einer beheizten Führungs- oder Umlenkrolle (24) gebildet ist, so dass der Sprühkleber im flüssigen Zustand von dem Auffangmittel (18) abgeführt wird.

9. Vorrichtung nach Anspruch 8, **dadurch gekennzeichnet, dass** das Auffangmittel (18, 26) in Überlappung mit der Bahn (4) hinter die Bahnebene reichend vorgesehen ist.

10. Vorrichtung nach Anspruch 8 oder 9, **dadurch gekennzeichnet, dass** das Auffangmittel (18) von einem beheizten Blech (20) gebildet ist.

11. Vorrichtung nach einem oder mehreren der vorstehenden Ansprüche 8, 9 oder 10, **dadurch gekennzeichnet, dass** die Bahn (4) in einem Förderabschnitt (16) der Maschine, in dem der Sprühkleberauftrag ausgeführt wird, zumindest randseitig nicht geführt ist.

## Claims

1. A method for producing disposable absorbent hygiene products (32), in particular in the form of underpants, in endless production in a high-speed manufacturing machine, wherein portions of flat material are joined together and glued by means of spray adhesive application by means of spray nozzles (6), and the spray adhesive (3) is sprayed onto a length of material (4) guided in a machine direction (2), and for forming a spray adhesive application that engages one edge (8) of the length of material (4), the spray nozzle (6) is positioned and oriented in a conveyor portion (16) of the machine in such a way relative to the edge (8) of the length of material (4) that its spray stream (10), which in particular widens in conical or fanlike form, extends past the edge (8) of the length of material (4), so that the length of material (4) is sprayed as far as the edge (8) with spray adhesive, and some of the spray adhesive is sprayed past the length of material (4), and in this conveyor portion (16), preferably at a slight spacing from one plane of the length of material (4), a collection means (18, 26) that engages the spray stream (10) is provided for the part of the spray adhesive that extends past the edge (8) of the length of material (4), so that spray adhesive sprayed past the edge (8) of the length of material (4) does not cause contamination of the machine, **characterized in that** the collection means (18) is embodied two-dimensionally and is heated or is formed by a heated guide or deflection roller (24), so that the spray adhesive is carried away from the collection means (18) in a liquid state.

2. The method of claim 1, **characterized in that** the collection means (18, 26) is provided such that it extends downstream of the plane of the length of material, overlapping length of material (4).

3. The method of claim 1 or 2, **characterized in that** the collection means (18) is formed by a heated metal plate (20).

4. The method of one or more of the foregoing claims, **characterized in that** the spray adhesive application is performed in a conveyor portion (16) of the machine in which the length of material (4) is not guided, at least at the edge.

5. The method of one or more of the foregoing claims, **characterized in that** the hygiene product (32) is embodied in the form of underpants, with a front abdominal portion (36) and a rear back portion (38) that are connected to one another by the manufacturer to form a continuous abdominal and back band in the transverse or hip circumference direction, with a hip opening, in side seam regions (33, 34) on both sides, that is closed in the hip circumference direction, and having a crotch portion (42) that has an absorption body (40) and extends in a longitudinal direction between the abdominal portion (36) and the back portion (38) and overlaps them, and **in that** it is undetachably joined in the overlapping region to the abdominal portion (36) and to the back portion (38) by means of spray adhesive, and the crotch portion (42), the abdominal portion (36) and the back portion (38) form the portions of material that are to be joined together, and the length of material (4) guided in the machine direction (2) and acted upon peripherally by spray adhesive forms the crotch portions (42).

6. The method of claim 5, **characterized in that** the length of material (4) forming the crotch portions (42) is cut, after spray adhesive has been sprayed on, transversely to the machine direction (2), forming successive separated crotch portions (42) which are then rotated by 90 degrees and are made to overlap with a length of material (58, 60) forming the back portions (36) and the abdominal portions (38), respectively, both lengths of material being conveyed in a machine direction (44) and joined adhesively to them.

7. A hygiene product (32) in the form of underpants, with a front abdominal portion (36) and a rear back portion (38) that are connected to one another by the manufacturer to form a continuous abdominal and back band in the transverse or hip circumference direction, with a hip opening, in side seam regions (33, 34) on both sides, that is closed in the hip circumference direction, and having a crotch portion (42) that has an absorption body (40) and extends in a longitudinal direction (37) between the abdominal portion (36) and the back portion (38) and overlaps them and undetachably joined in the overlapping region to the abdominal portion (36) and to the back portion (38) by means of spray adhesive, and the spray adhesive connection is embodied between a longitudinal peripheral portion (12) of the crotch portion (42) and the abdominal portion (36) and back portion (38), the product being obtained by a method having the features of the preamble to claim 1 and **characterized in that** the spray adhesive connection is embodied in the transverse direction (39) as far as the outermost longitudinal edge (8) of the crotch portion (42), **in that** the length of material (4) guided in the machine direction (2) and acted upon peripherally by spray adhesive forms the crotch portions (42, so that no longitudinal edges (8) of the crotch portion (42) can creep upward from the abdominal portion (36) and the back portion (38) and lead to skin irritations.

8. An apparatus for performing the method of one or more of the foregoing claims 1-6, wherein in a conveyor portion of the high-speed manufacturing machine, in which the spray adhesive application is performed, a spray nozzle (6) is positioned and oriented in such a way relative to the edge (8) of the length of material (4) that its spray stream (10), which in particular widens in conical or fanlike form, extends past the edge (8) of the length of material (4) to be sprayed, so that the length of material (4) is sprayed as far as the edge (8) with spray adhesive, and some of the spray adhesive is sprayed past the length of material (4), and in this conveyor portion (16), preferably at a slight spacing from one plane of the length of material (4), a collection means (18, 26) that engages the spray stream (10) is provided for the part of the spray adhesive that extends past the edge (8) of the length of material (4), so that spray adhesive sprayed past the edge (8) of the length of material (4) does not cause contamination of the machine, **characterized in that** the collection means (18) is embodied two-dimensionally and is heated or is formed by a heated guide or deflection roller (24), so that the spray adhesive is carried away from the collection means (18) in a liquid state.

9. The apparatus of claim 8, **characterized in that** the collection means (18, 26) is provided such that it extends downstream of the plane of the length of material, overlapping length of material (4).

10. The apparatus of claim 8 or 9, **characterized in that** the collection means (18) is formed by a heated metal plate (20).

11. The apparatus of one or more of the foregoing claims 8, 9 or 10, **characterized in that** in a conveyor portion (16) of the machine in which the spray adhesive application is performed, the length of material (4) is not guided, at least peripherally.

## Revendications

1. Procédé de fabrication en continu d'articles hygiéniques (32) absorbants à usage unique, en particulier sous forme de culottes, dans une machine de fabrication à grande vitesse, dans lequel des portions en matériau plat sont assemblées et collées entre elles par application de colle de pulvérisation au moyen de buses de pulvérisation (6), la colle de pulvérisation (3) étant projetée sur une bande (4) menée dans une direction de machine (2), dans lequel, pour former un dépôt de colle de pulvérisation couvrant un bord (8) de la bande (4), la buse de pulvérisation (6) est positionnée et orientée, dans une section de transport (16) de la machine, par rapport au bord (8) de la bande (4) de telle sorte que son jet de projection (10) s'élargissant en particulier en forme de cône ou d'éventail dépasse le bord (8) de la bande (4), de sorte que la bande (4) est pulvérisée de colle de pulvérisation jusqu'au bord (8) et qu'une partie de la colle de pulvérisation manque la bande (4), et dans lequel un moyen de réception (18, 26) qui reçoit le jet de projection (10) et est destiné à la partie de la colle de pulvérisation qui dépasse le bord (8) de la bande (4) est prévu dans cette section de transport (16), de préférence à une faible distance par rapport à un plan de la bande (4), de sorte que de la colle de pulvérisation qui manque le bord (8) de la bande (4) n'entraîne pas des salissures de la machine, **caractérisé par le fait que** ledit moyen de réception (18) est réalisé en nappe et chauffé ou est formé d'une poulie de guidage ou de renvoi (24) chauffée de sorte que la colle de pulvérisation est évacuée en état liquide dudit moyen de réception (18).

2. Procédé selon la revendication 1, **caractérisé par le fait que** le moyen de réception (18, 26) est prévu en recouvrement avec la bande (4), tout en s'étendant jusque derrière le plan de la bande.

3. Procédé selon la revendication 1 ou 2, **caractérisé par le fait que** ledit moyen de réception (18) est formé par une tôle (20) chauffée.

4. Procédé selon l'une ou plusieurs des revendications précédentes, **caractérisé par le fait que** l'application de colle de pulvérisation est réalisée dans une section de transport (16) de la machine où la bande (4) n'est pas guidée, au moins sur le bord.

5. Procédé selon l'une ou plusieurs des revendications précédentes, **caractérisé par le fait que** ledit article hygiénique (32) est réalisé sous forme de culotte et avec une portion ventrale avant (36) et une portion dorsale arrière (38) qui, pour la formation d'une bande ventrale et dorsale continue dans le sens transversal ou dans le sens du tour de hanches, avec une ouverture de hanche fermée dans le sens du tour de hanches, sont reliées entre elles par le fabricant au niveau de zones de couture latérale (33, 34) situées de part et d'autre, ainsi qu'avec une portion d'entrejambe (42) présentant un corps absorbant (40), qui s'étend dans une direction longitudinale entre la portion ventrale (36) et la portion dorsale (38) et recouvre celles-ci, et qu'elle est assemblée de manière inamovible, au moyen de colle de pulvérisation, dans la zone de recouvrement, sur la portion ventrale (36) et sur la portion dorsale (38), les portions d'entrejambe (42), ventrale (36) et dorsale (38) formant les portions de matériau à assembler, et la bande (4) qui est guidée dans la direction de machine (2) et qui, sur le bord, est revêtue de colle de pulvérisation formant les portions d'entrejambe (42).

6. Procédé selon la revendication 5, **caractérisé par le fait que** la bande (4) formant les portions d'entrejambe (42) est coupée transversalement à la direction de machine (2) après avoir appliqué par pulvérisation la colle de pulvérisation, de sorte que des portions d'entrejambe (42) successives individualisées sont formées qui, ensuite, sont tournées de 90 degrés et sont mises en recouvrement avec une bande (58) formant les portions dorsales (38) et avec une bande (60) formant les portions ventrales (36), transportées toutes les deux dans une direction de machine (44), et qui sont reliées par collage à celles-ci.

7. Article hygiénique (32) sous forme de culotte, comprenant une portion ventrale avant (36) et une portion dorsale arrière (38) qui, pour la formation d'une bande ventrale et dorsale continue dans le sens transversal ou dans le sens du tour de hanches, avec une ouverture de hanche fermée dans le sens du tour de hanches, sont reliées entre elles par le fabricant au niveau de zones de couture latérale (33, 34) situées de part et d'autre, et comprenant une portion d'entrejambe (42) présentant un corps absorbant (40), qui s'étend dans une direction longitudinale (37) entre la portion ventrale (36) et la portion dorsale (38) et recouvre celles-ci, et qui est assemblée de manière inamovible, au moyen de colle de pulvérisation, dans la zone de recouvrement, sur la portion ventrale (36) et sur la portion dorsale (38), la liaison par colle de pulvérisation étant réalisée entre une portion de bord longitudinal (12) de la portion d'entrejambe (42) et les portions ventrale (36) et dorsale (38), obtenu par un procédé ayant les caractéristiques du préambule de la revendication 1 et **caractérisé par le fait que** la liaison par colle de pulvérisation est réalisée dans le sens transversal (39) jusqu'au bord longitudinal (8) extrême de la portion d'entrejambe (42) **par le fait que** la bande (4) qui est guidée dans la direction de machine (2) et qui, sur le bord, est revêtue de colle de pulvérisation forme les portions d'entrejambe (42), de sorte qu'aucun des bords longitudinaux (8) de la portion d'entrejambe (42) ne peut se soulever de la portion ventrale (36) ou de la portion dorsale (38) et entraîner des irritations de la peau.

8. Dispositif de mise en oeuvre du procédé selon l'une ou plusieurs des revendications précédentes 1 à 6, dans lequel, dans une section de transport de la machine de fabrication à grande vitesse, où l'application de colle de pulvérisation est réalisée, une buse de pulvérisation (6) est positionnée et orientée par rapport au bord (8) de la bande (4) à pulvériser de telle sorte que son jet de projection (10) s'élargissant en particulier en forme de cône ou d'éventail dépasse le bord (8) de la bande (4), de sorte que la bande (4) est pulvérisée de colle de pulvérisation jusqu'au bord (8) et qu'une partie de la colle de pulvérisation manque la bande (4), et dans lequel un moyen de réception (18, 26) qui reçoit le jet de projection (10) et est destiné à la partie de la colle de pulvérisation qui dépasse le bord (8) de la bande (4) est prévu dans cette section de transport (16), de préférence à une faible distance par rapport à un plan de la bande (4), de sorte que de la colle de pulvérisation qui manque le bord (8) de la bande (4) n'entraîne pas des salissures de la machine, **caractérisé par le fait que** ledit moyen de réception (18) est réalisé en nappe et chauffé ou est formé d'une poulie de guidage ou de renvoi (24) chauffée de sorte que la colle de pulvérisation est évacuée en état liquide dudit moyen de réception (18).

9. Dispositif selon la revendication 8, **caractérisé par le fait que** ledit moyen de réception (18, 26) est prévu en recouvrement avec la bande (4), tout en s'étendant jusque derrière le plan de la bande.

10. Dispositif selon la revendication 8 ou 9, **caractérisé par le fait que** ledit moyen de réception (18) est formé par une tôle (20) chauffée.

11. Dispositif selon l'une ou plusieurs des revendications précédentes 8, 9 ou 10, **caractérisé par le fait que**, dans une section de transport (16) de la machine où l'application de colle de pulvérisation est réalisée, la bande (4) n'est pas guidée, au moins sur le bord.
